# EUROPEAN PATENT SPECIFICATION

(11) **EP 3 594 174 B1**
(45) Date of publication and mention of the grant of the patent: **31.03.2021**
(21) Application number: 19184191.5
(22) Date of filing: 03.07.2019
(51) Int. Cl.: B67D 1/00, B01F 3/04, F28D 7/10, B01F 5/06

(54) **IN-LINE CARBONATOR WITH DISINFECTION PROPERTIES AND BEVERAGE DISPENSER HAVING SUCH DEVICE**
FLUSSKARBONISATIONSVORRICHTUNG MIT DESINFEKTIONSEIGENSCHAFTEN UND GETRÄNKESPENDER MIT SOLCH EINER VORRICHTUNG
DISPOSITIF DE CARBONISATION DE TYPE FLUX PRÉSENTANT DES PROPRIÉTÉS DE DÉSINFECTION ET DISTRIBUTEUR DE BOISSONS DOTÉ D'UN TEL DISPOSITIF

(30) Priority: 11.07.2018 EP 18182943
(43) Date of publication of application: 15.01.2020
(73) Proprietor: RIPRUP Company S.A., St. Peter Port GY1 WQ (GG)
(72) Inventor: Bissen, Monique, 75175 Pforzheim (DE); Schucker, Josef, 6622 Ronco Sopra Ascona (CH)
(74) Representative: Wittmann, Günther

(56) References cited:
- US-A- 3 339 805
- US-A- 3 761 066
- US-A1- 2016 216 045

## Description

The present invention discloses a flow-type carbonization device with improved disinfection properties and a beverage dispenser having such flow-type carbonization device. A beverage dispenser outputs a beverage, such as water, into a glass or bottle of a user. Some users prefer carbonized beverage, such as carbonized water. Since water is supplied from a tap, a tank or a canister to the water dispenser in a non-carbonized way, the beverage dispenser must comprise a carbonization device for delivering carbonized beverage.

### Prior art

A significant amount of beverage dispensers comprises a tank in which water is carbonized. The water has to stagnate in this tank for a significant amount of time. Stagnation is generally undesired, since germs may form during stagnation.

Flow-type carbonization based on Venturi nozzles is known, wherein a stream of carbon dioxide is introduced in a water stream.

WO 2012/123462 A1 discloses a flow-type carbonization apparatus.

EP 0 322 925 A2 discloses a nozzle for injecting gas into a liquid.

The prior art flow-type water carbonizers have a comparably low efficiency. Further, prior art flow-type carbonizers are time consuming to disinfect, since the Venturi nozzle imposes a high flow resistance on the disinfection fluid.

US 3,339,805 discloses a carbonated water carbonator dispenser including a handle, extended from a head, with a nozzle extended angularly from the lower forward surface of the head and with a spiral mixing element in a bore of the handle. The end of the bore in the inner end of the handle is provided with a closure plug that is threaded in the bore. The lower end of the pre-mixing chamber is provided with a closing plug that is threaded into the bore of the chamber. Water supplied through a passage enters the mixing chamber, where it enters at tangent causing a swirling action, and at the same time CO₂ enters through the passage discharging into the chamber through the jet nozzle.

### Summary of the invention

It is an object of the present invention to provide a flow-type carbonization device with an improved efficiency and a beverage dispenser having such carbonization device that is efficient to disinfect.

The object of the present invention is achieved by a flow-type carbonization apparatus according to claim 1and a beverage dispenser according to claim 12.

The present invention discloses a flow-type carbonization device comprising a first pipe and a second pipe. Beverage to be carbonized and carbon dioxide flows in the first pipe. Beverage not to be carbonized flows in the second pipe. At least one turbulence generation element is arranged in the first pipe. The turbulence generation element supports solving of carbon dioxide in the beverage. The turbulence generation element may split up bubbles of carbon dioxide into smaller bubbles such that the carbon dioxide is solved with a higher concentration in the beverage. In one embodiment, the beverage may be water. The first and second pipes are in thermal communication such that heat from a fluid (liquid) flowing in the second pipe heats the first pipe.

The flow-type carbonization device may be efficiently disinfected, since hot water may be passed through the second pipe causing that the second pipe and first pipe and the at least one turbulence generation element is heated, such that germs, virus or pathogens are killed. In one embodiment a fluid (liquid) having a temperature of approximately 60°C to 99°C can heat the second pipe in a time span of less than 5 min. to approximately 50° C or higher.

Further, the second pipe may transport beverage not to be carbonized such as water for brewing coffee or tea or still water. If the beverage not to be carbonized does not pass the at least one turbulence generation element, the beverage may be dispensed faster and with less pump power, since the turbulence generation element in the first pipe does not does not impose any flow resistance to the beverage flowing in the first pipe.

The first and second pipes may be arranged concentric. This arrangement ensures a suitable thermal coupling and reduces space requirements.

In one embodiment the first pipe is arranged around the second pipe. However, it is also conceivable that the second pipe is arranged around the first pipe. It is preferred to arrange the first pipe around the second pipe for positioning more turbulence generation elements and/or a larger flow restricting area of turbulence generation elements and/or more turbulence generation openings into the flow of the beverage in the first pipe.

The at least one turbulence generation element reduces the cross section of the first pipe. Thereby, pressure of the beverage is increased, when passing the turbulence generation element, causing carbon dioxide bubbles to be split up and to be solved by the beverage more efficiently.

A plurality of turbulence generation elements may be arranged apart serially in the flow of beverage in the first pipe. Alternatively or additionally, a plurality of turbulence generation openings is arranged apart radially on the turbulence generation element.

In a preferred embodiment a plurality of turbulence generation openings is arranged apart around the circumference of the turbulence generation element. Thereby, the flow of beverage is forced to the outer portion of the first pipe and the spaces between the turbulence generation elements from turbulence chambers having a turbulent flow, in which the bubbles of carbon dioxide are further split up and solved by the beverage.

In one embodiment the turbulence generation element has a generally circular cross section at its outer perimeter. The cross section of the turbulence generation element is arranged perpendicular to the axial direction of the first pipe and the direction of flow of beverage in the first pipe. At least one turbulence generation opening is formed by at least one recess at the outer perimeter of the turbulence generation element. Thereby, the flow of beverage is forced to the outer portion of the first pipe and the spaces between the turbulence generation elements form a turbulence chamber. Carbon dioxide bubbles are split up at the edges of the recess and solved in the beverage in the turbulence chamber.

In one embodiment, the recess may be formed by a flattened portion of the generally circular cross section of the turbulence generation element. The turbulence generation element blocks flow of any fluid between the outer wall of the second pipe to the inner wall of the first pipe, except at the at least one recess at the outer perimeter of the turbulence generation element.

In one embodiment the recess in the turbulence generation element may be formed by a first wall orthogonal to the radius of the first pipe and at least one second wall perpendicular to the first wall.

In one embodiment a plurality of turbulence generation elements is arranged in serial relationship forming turbulence chambers between the opposite turbulence generation element, the outer cylindrical wall of the second pipe and the inner cylindrical wall of the first pipe.

The distance in axial direction of the first tube between two turbulence generation elements arranged in serial relationship may be at least two times of the thickness of the turbulence generation element in axial direction of the first pipe. The distance in axial direction of the first pipe between two turbulence generation elements arranged in serial relationship may range between approximately two to approximately three times of the thickness of the turbulence generation element in axial direction of the first pipe. The distance in axial direction of the first pipe between two turbulence generation elements arranged in serial relationship is at least two times the difference of the inner diameter of the first pipe and the outer diameter of the second pipe. The distance in axial direction of the first pipe between two turbulence generation elements arranged in serial relationship is approximately two times to approximately three times the difference of the inner diameter of the first pipe and the outer diameter of the second pipe. The width of the recess of the turbulence generation element orthogonal to the radius of the first pipe ranges between approximately 75% to approximately 125% of the thickness of the turbulence generation element in axial direction of the first pipe. The maximum height of the recess in radial direction of the first pipe may range from approximately 0.5% to approximately 1.5% of the thickness of the turbulence generation element in axial direction of the first pipe.

A flow-type carbonization apparatus according to the present invention comprising a carbonization controller, the flow-type carbonization device as described above and at least one control valve adapted to direct a fluid to the first pipe and/or second pipe. The controller may be an embedded computer on which a software is running. The control valve may be a Y-valve. The carbonization controller is adapted in a first operation mode of the flow-type carbonization apparatus to switch the at least one control valve such that beverage to be carbonized is directed to the first pipe and to switch the at least one control valve such that beverage not to be carbonized is directed to the second pipe.

The carbonization controller is adapted in a second operation state to switch the control valve such to direct a disinfection fluid through the second pipe. In one embodiment, the disinfection fluid (liquid) may be water heated to a range of 60°C to 99°C, preferably between 75°C and 85°C. The hot fluid flowing through the second pipe heats the first pipe, the turbulence generation elements, the recesses therein and other elements in the first pipe such that germs, virus and pathogens are destroyed. The disinfection fluid and/or hot fluid may flow through the second pipe, until the first pipe is disinfected and/or sterilized. Thereafter, the carbonization controller may also direct sterilizing fluid through the first pipe for removing the destroyed germs, virus or pathogens or the like.

The invention also discloses a beverage dispenser comprising the flow-type carbonization apparatus disclosed above. The beverage dispenser comprises a liquid flow valve and/or a liquid pump adapted to control the flow of beverage through the flow-type carbonization device. The beverage dispenser may further comprise a gas valve and/or a gas pump adapted to control the flow of gas into a gas inlet portion for supplying the beverage with carbon dioxide. The controller may be adapted to control the liquid flow valve and/or liquid pump and the gas valve and/or the gas pump. The controller may control the liquid flow valve and/or liquid pump and the gas valve and/or gas pump such that gas is fed into the gas inlet portion during flow of the beverage through the flow-type carbonization device.

In one embodiment the gas inlet portion may comprise at least a first gas injector and a second gas injector for injecting gas into the gas inlet section, wherein the first gas injector causes a first gas output flow and the second gas injector causes a second gas output flow, wherein the second gas output flow is at least 50% larger, preferably 70% larger, more preferred between 80% and 120%, most preferred at least 80 % larger than the first gas flow. Thereby, the amount of gas injected into the liquid can be controlled over a wider range without requiring additional process time for carbonization. The flow-type carbonization device may further comprise a carbonization controller adapted to control the first gas injector and the second gas injector, wherein if a low quantity of gas shall be fed into the liquid, only the first gas injector is activated, if a medium quantity of gas shall be fed into the liquid, only the second gas injector is activated and if a high quantity of gas shall be fed into the liquid the first gas injector and the second gas injector are activated. It is to be understood that the medium quantity of gas is larger than the low quantity of gas and the high quantity of gas is higher than the medium quantity of gas. The amount of carbon dioxide injected into the liquid may also be controlled by the time of activation of the first and/or second gas injector.

Preferably the flow of beverage is less than 1 I per minute, preferably between 0.5 I per minute to 1 I per minute. If the beverage to be carbonized has a temperature of 2°C the carbon dioxide concentration of approximately 5 g/l can be achieved with the present carbonization device. If the beverage has a temperature of 8°C a carbon dioxide concentration of approximately 4 g/l can be achieved with the inventive flow-type carbonization device. This corresponds to an efficiency of approximately 60%. The beverage fed through the carbonization device may have a pressure from approximately 3 bar to approximately 4 bar. Between the carbon dioxide tank and the first gas injector and/or the second gas injector a pressure reducing valve, particularly a pressure regulating valve can be located to control the pressure of the carbon dioxide in a controlled range. A preferred carbon dioxide pressure at the inlet of the first and/or second gas injector is approximately 5 bar to approximately 6 bar.

The beverage dispenser may further comprise a tempering device arranged downstream of the gas injection portion and upstream of the turbulence device. Preferably, the tempering device is a flow-type tempering device. The liquid flow in the tempering device is not laminar but rather meander shaped which supports reducing the size of the carbon dioxide bubbles and thus solving the carbon dioxide in the liquid, such as water.

In one embodiment the amount of gas injected may be time modulated by activating a gas injector in the gas inlet portion over a time period varying depending on the set concentration of carbon dioxide in the water independent of the configuration of the turbulence generation elements.

The invention also discloses an alternative flow-type carbonization device (flow-type carbonization section) comprising a liquid inlet for feeding pressurized liquid, a liquid outlet for discharging carbonized liquid, a gas inlet portion located downstream of the liquid inlet and a turbulence section located downstream of the gas inlet portion through which the pressurized liquid flows, when gas flows through the gas inlet portion. The turbulence section is in fluid communication with the liquid inlet and the liquid outlet. The turbulence section comprises at least one turbulence element having an outer pipe portion and an inner pipe portion. The outer pipe portion is partially closed by a dividing wall and an inner pipe portion extends from the partially open dividing wall. The inner pipe portion extends within the outer pipe portion. The inner pipe portion and the outer pipe portion are connected by the dividing wall. A recess is formed between a portion of the inner pipe portion and the outer pipe portion. The inner pipe portion and the outer pipe portion are in flow communication with the liquid inlet and the liquid outlet. The alternative flow-type carbonization device (section) may be part of the above described flow-type carbonization apparatus and/or beverage dispenser.

The inner pipe portion may extend upstream from the dividing wall into the recess formed by the outer pipe portion. Thereby, water flowing from a chamber formed by the outer pipe portion is formed into the inner pipe portion having a smaller diameter than the outer pipe portion. The inventors of the present invention assume without wishing to be bound to a specific theory that the carbon dioxide bubbles are fragmented at the edge of the orifice of the inner pipe portion extending upstream into the outer pipe portion and solved by the liquid.

In use the beverage flows through the inner pipe portion into a chamber formed by the outer pipe portion. Thereby, a part of the liquid injected by the inner pipe portion is directed to the recess formed between the inner pipe portion, the outer pipe portion and the dividing wall. The inventors of the present invention assume without wishing to be bound to a specific theory that at the edge of the orifice of the inner pipe portion protruding into the chamber formed by the outer pipe portion the carbon dioxide bubbles are fragmented and solved in a more efficient way in the beverage.

The inner pipe portion may extend downstream into the dividing wall into the recess formed by the outer pipe portion. Further, the recess around the inner pipe portion causes a turbulent flow supporting solving of the carbon dioxide in the liquid.

The outer pipe portion extends further from the separating wall than the inner pipe portion, such that the outer pipe portion may form a chamber in which the beverage flows from the inner pipe portion and/or from which the beverage may flow into the inner pipe portion.

In one embodiment, the turbulence section comprises a plurality of turbulence elements in serial connection. The beverage flows from the liquid inlet through the plurality of turbulence elements in a serial flow connection to the liquid outlet.

In a portion of the turbulence section the dividing walls of two adjacent turbulence elements may be located adjacent to each other. In another portion of the turbulence section the end portion of the outer pipe portions of two adjacent turbulence elements may be located adjacent to each other, wherein the end portions of the inner pipe portions face each other. The two outer pipe portions form a chamber into which the two inner pipe portions extend at opposite sides of the chamber from the respective dividing wall.

The outer pipe portion and the dividing wall of the turbulence element form a cylinder wherein the inner pipe portion forms an opening in the dividing wall.

The turbulence section may comprise a plurality of chambers that are in serial flow communication with an inlet of the turbulence section and an outlet of the turbulence section. The chambers are formed by the outer pipe portions. The chambers are separated by the dividing walls. Each inner pipe portion extends through a dividing wall into the adjacent chambers. Recesses are formed around an inner pipe portion extending into the outer pipe portion. Since a plurality of chambers and inner pipe portions are arranged in serial fluid communication, the efficiency of the flow-type carbonization device is increased significantly. In one embodiment three to four chambers are preferred. Generally, a fifth chamber does not increase the achieved carbon dioxide concentration in the water significantly.

The distance between two orifices of opposing inner pipe portions facing each other may correspond to approximately 50% to approximately 150%, preferably to approximately 70% to approximately 125%, more preferred to approximately 100% to approximately 120% of the inner diameter of the outer pipe portion. The distance between two orifices of opposing inner pipe portions facing each other may correspond to approximately 50% to approximately 150%, preferably to approximately 75% to approximately 125%, more preferred to approximately 85% to approximately 115% of the length of a flow channel formed by the inner pipe portion extending in a first chamber and a second inner pipe portion extending in a second chamber adjacent to the first chamber. The diameter of the inner pipe portion may correspond to approximately 5% to approximately 30%, preferably to approximately 10% to approximately 25%, more preferred to approximately 15% to approximately 20% of the diameter of the outer pipe portion. The thickness of the wall of the inner pipe portion may correspond to approximately 50% to approximately 100%, preferably to approximately 65% to approximately 85%, more preferred to approximately 70% to approximately 75% of the diameter of the inner pipe portion. The inner pipe portion may extend from the dividing wall approximately 50% to approximately 400%, preferably approximately 100% to approximately 300%, more preferred approximately 150% to approximately 250% of the diameter of the inner pipe into the chamber.

The inner pipe portion has to be sharp edged at the orifice. Preferably, the orifice of the inner pipe portion is manufactured by drilling.

The distance between the orifices of opposing inner pipe portions facing each other ranges approximately from 3.5 mm to approximately 12 mm, preferably from approximately 4.5 mm to approximately 10 mm, more preferred from approximately 6 mm to approximately 8 mm. The length of a flow channel formed by a first inner pipe portion extending in a first chamber and a second inner pipe portion extending in a second chamber adjacent to the first chamber ranges from approximately 3.5 mm to approximately 12 mm, preferably from approximately 4.5 mm to approximately 10 mm, more preferred from approximately 6 mm to approximately 8 mm. The diameter of the inner pipe portion may range from approximately 0.5 mm to approximately 3 mm, preferably from approximately 0.7 mm to approximately 2 mm, more preferred from approximately 1 mm to approximately 1.5 mm. The thickness of the wall of the inner pipe portion ranges from approximately 0.3 mm to approximately 1.5 mm, preferably from approximately 0.5 mm to approximately 1 mm, more preferred from approximately 0.7 mm to approximately 0.8 mm. The inner pipe portion may extend from the dividing wall approximately 1 mm to approximately 3 mm, preferably approximately 1.5 mm to 2.5 mm, more preferred approximately 1.7 mm to approximately 2.2 mm into the chamber. The inner diameter of the outer pipe portion ranges between approximately 4 mm to approximately 10 mm, preferably between approximately 4 mm to approximately 8 mm, most preferred between approximately 5 mm to approximately 7 mm.

### Short description of the drawings

The invention is now described in further detail with reference to the accompanying drawings showing a non-limiting embodiment of the present invention, wherein:
Figure 1 is a schematic diagram of components of a beverage dispenser;
Figure 2 is a schematic sectional view of a turbulence device according to a first embodiment of the present invention; and
Figure 3 is a schematic sectional view of a turbulence generation element according to the first embodiment of the present invention; and
Figure 4 is a schematic sectional view of a turbulence device according to a second embodiment of the present invention.

### Detailed description of the invention

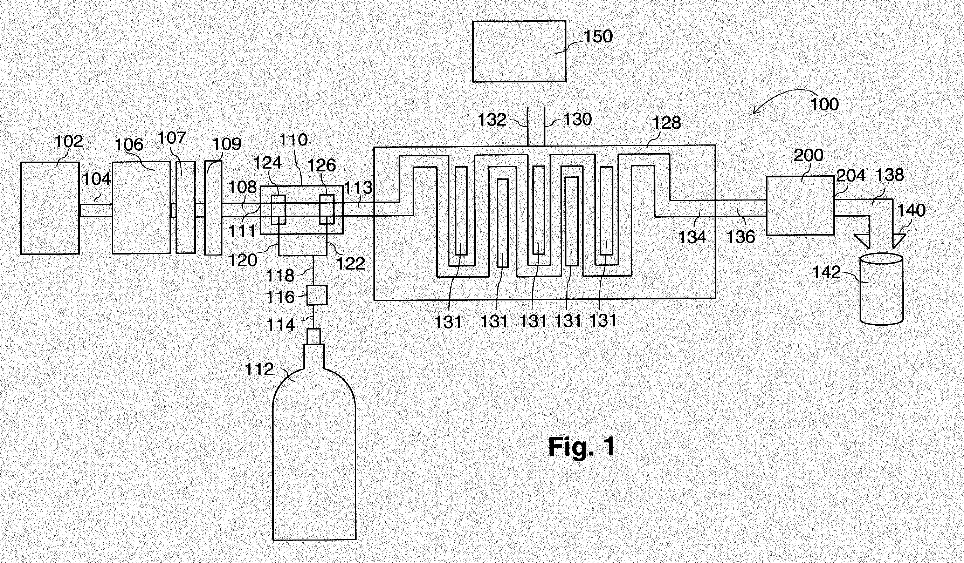

Reference is made to figure 1, showing a schematic view of a beverage dispenser 100 employing the present invention. The invention is described with reference to a water dispenser 100, but it is to be understood that the invention can be applied to any type of beverage dispenser. Reference numeral 102 indicates a water source. The water source may be a tap, a tank, a canister or the like. The water source 102 is connected by a pipe 104 to a pump 106. The pump 106 supplies water with a pressure of approximately 3 bar to approximately 4 bar into a pipe 108 connected to a gas inlet portion 110. The gas inlet portion 110 comprises a liquid inlet 111 for receiving pressurized water. The gas inlet portion 110 comprises a first gas injector 124 and a second gas injector 126. The second gas injector 126 can supply approximately twice as much carbon dioxide to the water flowing through the gas inlet portion as compared to the first gas injector 124.

The opening of the second gas injector may have a larger area as the opening of the first gas injector. The area of opening of the second gas injector may be two times larger as the area of the opening of the first gas injector. The area of the opening of the second gas injector may be at least 50 % larger, preferably 70 % larger, more preferred between 80 % and 120 % larger, most preferred at least 80 % larger than the area of the opening of the first gas injector.

The water dispenser 100 comprises a carbon dioxide bottle 112 connected by a pipe 114 to a pressure reducing valve or pressure regulating valve 116. The pressure reducing valve 116 supplies carbon dioxide with a pressure of approximately 5 bar to approximately 6 bar to a pipe 118. The pipe 118 branches into a first injector supply pipe 120 and a second injector supply pipe 122. The first injector supply pipe 120 is connected to the first gas injector 124 and the second injector supply pipe 122 is connected to the second gas injector 126.

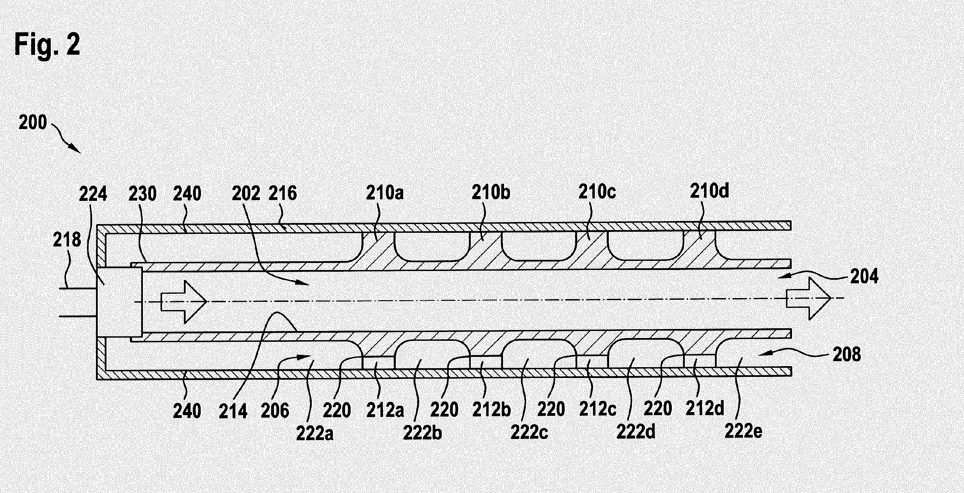

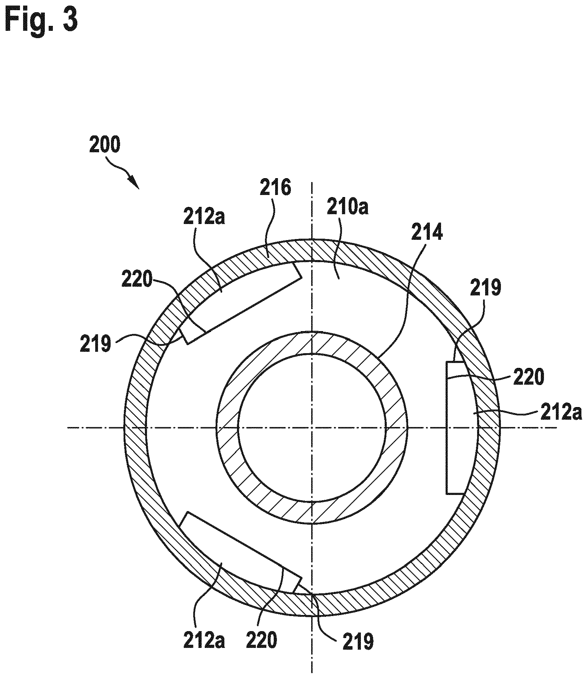

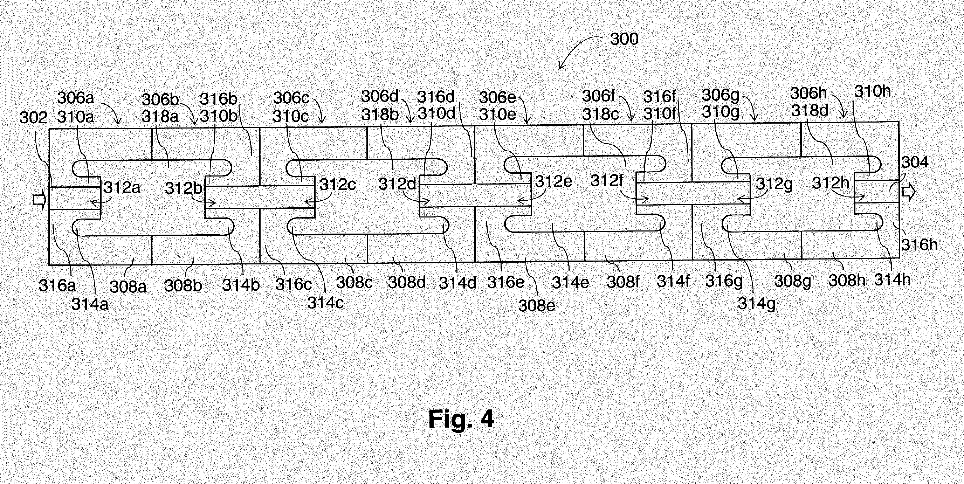

The gas inlet portion 110 is connected by an optional pipe 113 to a tempering device 128, i.e. a cooler. The water flows in the cooler through a meander-shaped pipe 134 which passes adjacent to cooling element 131. The cooling element 131 may comprise a Peltier element connected to a power supply 130, 132. The cooling element 131 may also be a heat exchanger through which a cooling media passes which is supplied by pipe 130 and discharged by pipe 132. The tempered water exits through an optional pipe 136 into a turbulence section 200 described in further detail with reference to figures 2 and 3 according to a first embodiment of the turbulence section 200 and with reference to figure 4 according to a second embodiment the turbulence section 300.

The turbulence section 200 comprises an outlet 208 for outputting a carbonized water to a pipe 138 to which a nozzle 140 is connected dispensing the carbonized water into a vessel 142 of a user.

The water dispenser 100 further comprises a flow-type heater 107 arranged between the pipe 108 and a cleaning agent device 109 adapted to heat the water to a temperature of at least 70°, preferably 80°, more preferred 90°. The water acts as a sterilizing fluid, to which cleaning agents may be added by the cleaning agent device 109. Therefrom, the cleaning fluid flows downstream to the gas inlet portion 110, the flow-type tempering device 128 and through the turbulence section 200 for sterilizing these components, if a controller 150 switches the water dispenser 100 from a beverage dispensing mode to a cleaning mode.

Reference is made to figures 2 and 3 showing a first and preferred embodiment of the turbulence section 200. The turbulence section 200 comprises a first inlet 206 through which water to be carbonized enters the turbulence section 200. The turbulence section 200 comprises a first outlet 208, through which carbonated water exits the turbulence section 200. Further, the turbulence section 200 comprises a second inlet 202 through which water not to be carbonated enters, and a second outlet 204, through which water not to be carbonated exits from the turbulence section 200.

Between the first inlet 206 and the first outlet 208 a plurality of turbulence generation elements 210a, 210b, 210c, 210d are arranged. The plurality of turbulence generation elements 210a, 210b, 210c, 210d are formed integrally with a second pipe 214 formed between the second inlet 202 and the second outlet 204. Around the turbulence generation elements 210a, 210b, 212c, 210d a first pipe 216 is extending connecting the first inlet 206 with the first outlet 208.

As can be seen in figures 2 and 3 the turbulence generation elements 210 are generally solid and extend from the second pipe 214 to the first pipe 216. At the outer perimeter of the generally circular turbulence generation element 210 a plurality of turbulence generation openings 212a, 212b, 212c, 212d are arranged. The turbulence generation openings 212a may be arranged along the perimeter of the turbulence generation elements 210a. In the embodiment shown in figures 3, three turbulence generation openings 212a are arranged along (around) the perimeter of the turbulence generation element 210a. In another embodiment more turbulence generation openings or less turbulence generation openings may be arranged along the perimeter of the turbulence generation element 210a, such as two turbulence generation openings, four turbulence generation openings or more turbulence generation openings.

As can be seen in figure 3 each turbulence generation opening may comprise a first portion 220 extending generally perpendicular to the radial direction of the turbulence generation element. Perpendicular to the first portion 220 of the turbulence generation opening a second portion 219 may be arranged.

As can be seen in figure 2, a plurality of turbulence generation elements 210a, 210b, 210c, 210d and/or a plurality of turbulence generation openings 212a, 212b, 212c, 212d may be arranged in serial relationship in the flow direction indicated by the arrows in figure 2. The turbulence generation elements 210a, 210b, 210c, 210d may be spaced apart to form turbulence chambers 222a, 222b, 222c, 220d, 220e in front of the turbulence generation elements 210a, between the turbulence generation elements 210a, 210b, 210c, 210d and/or behind the turbulence generation element 210d in the flow direction of the water to be carbonized. Without wishing to be bound to be a particular theory, the inventors of the present invention assume that carbon dioxide bubbles are split up at the turbulence generation openings 212a, 212b, 212c, 212d and solved in the water. Further, at the turbulence generation openings 212a, 212b, 212c, 212d a higher pressure is generated, resulting in that the carbon dioxide bubbles are solved in the beverage and water, respectively. Further, the turbulence generated in the turbulence chambers 222a, 222b, 222c, 222d, 222e results in that the beverage and water, respectively solves the carbon dioxide.

The thickness of the turbulence generation elements 210a, 210b, 210c, 210d may range between approximately 1 mm to 3 mm. The distance between two turbulence generation elements 210a, 210b, 210c, 210d may range between 3 to 7 mm. The inner diameter of the first pipe 216 may range between 7 and 10 mm, and the outer diameter of the second pipe may range between 4 and 6 mm.

In the embodiment according to figures 2 and 3 the first pipe 216 and the second pipe 214 are drawn to be concentric. This does not have to be the case, the first pipe 216 and the second pipe 214 may be coupled thermally by any suitable means, such as a heat conductor, for example copper, or a heat pipe.

The turbulence section 200 is connected to a valve 224. The inlet 218 of the valve 224 is connected to the pipe 136 transporting beverage and water, respectively from the flow-type water tempering device 128. The valve 224 is operatively connected to the controller 150. If the controller 150 determines that water is not to be carbonized, the water entering the valve 224 at the inlet 218 is passed to a second outlet pipe 230 of the valve 224 and enters the second inlet 202 of the turbulence section 200. Water not to be carbonized may be water for preparing tea, coffee or still water. The water flowing in the second pipe 214 does not pass any turbulence elements and no carbon dioxide has been injected by the first and second injection valves 124, 126. Therefore, the water exits the second outlet 204 without being carbonized and enters the nozzle 140.

If the controller 150 determines that water is to be carbonized, carbon dioxide is injected by the first and/or second injection valve 124, 126. Further, the valve 224 is switched such that beverage and water, respectively entering the inlet 218 of the valve 224 is passed to a first outlet pipe 240 of the valve 224, wherein the first outlet pipe 240 is connected to the first inlet 206 of the turbulence device 200. The beverage and water, respectively passes the turbulence generation elements 210a, 210b, 210c, 210d comprising the turbulence generation openings 212a, 212b, 212c, 212d, respectively, in which the carbon dioxide bubbles are split up and solved by the beverage and water respectively, as described above.

In a first step of a disinfection operation mode the controller 150 may pass water heated by the flow-type water heater 107 and optionally supplemented by the cleaning agent dispensing device 109 to the second pipe 214 by switching the valve 224 such that the hot water is flowing from the inlet 218 of the valve 224 to the second outlet pipe 230. The hot water enters the second inlet and heats the second pipe 214 and the turbulence generation elements 210a, 210b, 210c, 210d and thus also the first pipe 216. Thereby, the turbulence elements 200 is effectively disinfected and/or sterilized. As soon as all germs have been destroyed in the turbulence chambers 222a, 222b, 222c, 220d, 220e and the turbulence openings 212a, 212b, 212c, 212d, the controller 150 may switch the valve 224 in a second step such that hot water flows from the inlet 218 of the valve 224 to the first pipe outlet 240 and thus into the first inlet 206 for removing the destroyed germs, pathogens and virus from the turbulence chambers 222a, 222b, 222c, 220d, 222e and the turbulence generation opening 212a, 212b, 212c, 212d.

The first embodiment of the turbulence chamber 200 allows effective flow-type carbonization by a flow-type turbulence section 200 by turbulence chambers 222a, 222b, 222c, 220d, 220e and turbulence openings 212a, 212b, 212c, 212d. Although the turbulence generation openings restrict the flow of a liquid, the turbulence section can be effectively disinfected and/or sterilized, since hot sterilizing liquid may be passed between the second inlet 202 and the second outlet 204 and since the liquid flowing from the second inlet 202 to the second outlet 204 is in thermal communication with the turbulence generation elements 210a, 210b, 210c, 210d and the first pipe 216.

Reference is made to figure 4 showing a schematic cut away view of a turbulence section (flow-type carbonization device) 300 according to a third embodiment of the present invention. The turbulence section 300 comprises essentially four chambers 318a, 318b, 318c, 316d formed by outer pipe portions 308a-308h of a plurality of turbulence elements 306a-306h.

Into the first chamber 318a an inner pipe portion 310a of a first turbulence element 306a extends. Between the outer pipe portion 308a and the inner pipe portion 310a a recess 314a is formed. Between the outer pipe portion 308a and the inner pipe portion 310a a dividing wall 316a is arranged. The outer pipe portion 308a and the dividing wall 316a may form a cylinder, wherein the inner pipe portion 310a extends through the dividing wall 316a. The inner pipe portion 310a forms a fluid passage, wherein the fluid enters through the orifice 312a of the inner pipe portion 310a into the first chamber 316a. The outer pipe section 308a of the first turbulence element 306a extends further in the downstream direction as the inner pipe portion 310a of the first turbulence element 306a. The flow direction is indicated in Fig. 4 by arrows.

Adjacent to the first turbulence element 306a a second turbulence element 306b is located. The second turbulence element 306b is shaped essentially the same way as the first turbulence element 306a. Thus, for the sake of brevity, the second turbulence element is not described detail. The second turbulence element also comprises an outer pipe portion 308b connected by a dividing wall 316b with an inner pipe portion 310b. The second turbulence element 306b is arranged such in the turbulence section 300 that an orifice 312b of the inner pipe portion 316b of the second turbulence element 306b faces the orifice 312a of the inner pipe portion 310a of the first turbulence element 306a. The inner pipe portion 310b of the second turbulence element 306b extends upstream into the first chamber 318a.

The fluid enters through an orifice 312b in the inner pipe portion 310b of the second turbulence element 306b. The outer pipe portion 308b extends further from the divisional wall 316b in the upstream direction as the inner pipe portion 310b. Between the outer pipe portion 308b of the second turbulence element 306b and the inner pipe portion 310b a recess 314b is formed.

The combination of first turbulence element 306a and second turbulence element 306b can form in one embodiment a turbulence section having a single chamber 318a.

For increasing the efficiency of a plurality of a chambers 318a-318d and a plurality of turbulence elements 306a-306h can be arranged in serial flow communication.

In the embodiment disclosed in figure 4, adjacent to the second turbulence element 306b a third turbulence element 306c is arranged. The third turbulence element 306c is shaped essentially the same way as the first turbulence element 306a. A divisional wall 316c of the third turbulence element 306c is arranged adjacent (face-to-face) to the divisional wall 316b of the second turbulence element 306b. Thus, the inner pipe portion 310c of the third turbulence element extends downstream into the chamber 318b formed by the outer pipe portion 308c of the third turbulence element 306c. The fluid flows through the passage formed by the inner pipe portion 312b of the second turbulence element and the inner pipe portion 312c of the third turbulence element 306c and enters through the orifice 312c of the inner pipe portion 310c of the third turbulence element 306c into the chamber 318b. Between the outer pipe portion 308c and the inner pipe portion 310c a recess 314c is formed.

Adjacent to the third turbulence element 306c a fourth turbulence element 306d is located. The fourth turbulence element 306d is shaped essentially the same way as the first turbulence element 306a. Thus, for the sake of brevity, the fourth turbulence element is not described detail. The fourth turbulence element also comprises an outer pipe portion 308d connected by a dividing wall 316d with an inner pipe portion 310d. The fourth turbulence element 306d is arranged such in the turbulence section 300 that an orifice 312d of the inner pipe portion 310d of the fourth turbulence element 306d faces the orifice 312c of the inner pipe portion 310c of the third turbulence element 306c. The inner pipe portion 310d of the fourth turbulence element 306d extends upstream into the second chamber 318b.

The fluid enters through an orifice 312d from the chamber 318b in the of the inner pipe portion 310d of the second turbulence element 306d. The outer pipe portion 308d extends further from the divisional wall 316b in downstream direction as the inner pipe portion 310d. Between the outer pipe portion 308d of the fourth turbulence element 306d and the inner pipe portion 310d a recess 314d is formed.

Adjacent to the fourth turbulence element 306d a fifth turbulence element 306e is arranged. The fifth turbulence element 306e is shaped essentially the same way as the first turbulence element 306a. A divisional wall 316e of the fifth turbulence element 306d is arranged adjacent (face-to-face) to the divisional wall 316d of the fourth turbulence element. Thus, the inner pipe portion 310e of the fifth turbulence element extends downstream into a third chamber 318c formed by the outer pipe portion 308e of the fifth turbulence element 306e. The fluid flows through the passage formed by the inner pipe portion 312d of the fourth turbulence element and the inner pipe portion 310e of the fifth turbulence element 306e and enters through the orifice 312e of the inner pipe portion 310e of the fifth turbulence element 306e into the chamber 318c. Between the outer pipe portion 308e and the inner pipe portion 310e a recess 314e is formed.

Adjacent to the fifth turbulence element 306e a sixth turbulence element 306f is located. The sixth turbulence element 306f is shaped essentially the same way as the first turbulence element 306a. The sixth turbulence element also comprises an outer pipe portion 308f connected by a dividing wall 316f with an inner pipe portion 310f. The sixth turbulence element 306f is arranged such in the turbulence section 300 that an orifice 312f of the inner pipe portion 316f of the sixth turbulence element 306f faces the orifice 312e of the inner pipe portion 310e of the fifth turbulence element 306e. The inner pipe portion 310f of the sixth turbulence element 306f extends upstream into the third chamber 318c.

Adjacent to the sixth turbulence element 306f a seventh turbulence element 306g is arranged. The seventh turbulence element 306g is shaped essentially the same way as the first turbulence element 306a. A divisional wall 316g of the seventh turbulence element 306g is arranged adjacent (face-to-face) to the divisional wall 316f of the sixth turbulence element. Thus, the inner pipe portion 310g of the seventh turbulence element extends downstream into a fourth chamber 318d formed by the outer pipe portion 308g of the seventh turbulence element 306g. The fluid flows through the passage formed by the inner pipe portion 312f of the sixth turbulence element 306f and the inner pipe portion 310g of the seventh turbulence element 306g and enters through the orifice 312g of the inner pipe portion 310g of the seventh turbulence element 306g into the fourth chamber 318d. Between the outer pipe portion 308g and the inner pipe portion 310g a recess 314g is formed.

Adjacent to the seventh turbulence element 306g an eighth turbulence element 306h is located. The eighth turbulence element 306h is shaped essentially the same way as the first turbulence element 306a. The eighth turbulence element 306h also comprises an outer pipe portion 308h connected by a dividing wall 316h with an inner pipe portion 310h. The eighth turbulence element 306h is arranged such in the turbulence section 300 that an orifice 312h of the inner pipe portion 316h of the eighth turbulence element 306h faces the orifice 312g of the inner pipe portion 310g of the seventh turbulence element 306g. The inner pipe portion 310h of the eighth turbulence element 306h extends downstream into the fourth chamber 318d.

The distance between the orifices 312a-312h of opposing inner pipe portions 310a-310h facing each other ranges approximately from 3.5 mm to approximately 12 mm, preferably from approximately 4.5 mm to approximately 10 mm, more preferred from approximately 6 mm to approximately 8 mm. The length of a flow channel formed by a first inner pipe portion 310a-310h extending in an upstream chamber 318a-318c and a second inner pipe portion 310a-310h extending in a downstream chamber 318b-318d adjacent to the first chamber ranges from approximately 3.5 mm to approximately 12 mm, preferably from approximately 4.5 mm to approximately 10 mm, more preferred from approximately 6 mm to approximately 8 mm. The diameter of the inner pipe portion 310a-310h may range from approximately 0.5 mm to approximately 3 mm, preferably from approximately 0.7 mm to approximately 2 mm, more preferred from approximately 1 mm to approximately 1.5 mm. The thickness of the wall of the inner pipe portion 310a-310h ranges from approximately 0.3 mm to approximately 1.5 mm, preferably from approximately 0.5 mm to approximately 1 mm, more preferred from approximately 0.7 mm to approximately 0.8 mm. The inner pipe portion 310a-310h may extend from the dividing wall 316a-316h approximately 1 mm to approximately 3 mm, preferably approximately 1.5 mm to 2.5 mm, more preferred approximately 1.7 mm to approximately 2.2 mm into the chamber. The inner diameter of the outer pipe portion 308a-308h ranges between approximately 4 mm to approximately 10 mm, preferably between approximately 4 mm to approximately 8 mm, most preferred between approximately 5 mm to approximately 7 mm.

The operation of the turbulence section is described below in more detailed. A fluid, in this embodiment the fluid comprising water and carbon dioxide, enters through the orifices 312a, 312c, 312e, 312g of the first, third, fifth and seventh turbulence element 306a, 306c, 306e, 306g into the respective chamber 318a, 318b, 318c, 318d. The inventors assume without wishing to be bound to a specific theory that at the orifice 312a, 312c, 312e, 312g the carbon dioxide bubbles are split up and distributed in the water and dissolve in the water. Further, the recess 314a, 314c, 314e, 314g around the inner pipe portion 310a, 310c, 310e, 310g forms a turbulent flow of the fluid in which the water can dissolve carbon dioxide in a particular efficient way.

Further, the recess 314a, 314c, 314e, 314g cause a particular turbulence flow in the chambers 318a, 318b, 318c, 318d contributing to solving carbon dioxide in water.

The fluid exits the chamber 318a, 318a, 318c, 318d by the orifice 312b, 312d, 312f, 312h of the inner pipe portion 310b, 310d, 310f, 310h of the second, fourth, sixth and eight turbulence element 306b, 306d, 306f, 306h, respectively. The inventors assume that at the edge of the orifice 312b, 312d, 312f, 312h the bubbles of carbon dioxide are divided and split up and dissolved more efficiently in the water. Further, the recess 314b, 314d, 314f, 314h between the outer pipe portion 306b, 306d, 306f, 306h and the inner pipe portion 316b, 316d, 316f, 316h increase the turbulence of the flow in the chamber 318a, 318b, 318c, 318d adding to the efficiency of the carbonization.

Preferably the flow of water through the turbulence section 300 is less than 1 I per minute, preferably between 0.5 I per minute to 1 I per minute. If the water to carbonite has a temperature of 2°C a carbon dioxide concentration of 5 g/l can be achieved with the present carbonization device. If the water has a temperature of 8°C a carbon dioxide concentration of 4 g/l may be achieved with the inventive flow-type carbonization device. This corresponds to an efficiency of approximately 60%. The water fed through the gas inlet portion 110 and/or the turbulence section 300 may have a pressure from approximately 3 bar to approximately 4 bar.

## Claims

1. A flow-type carbonization apparatus, comprising:
- a carbonization controller (150);
- a flow-type carbonization device including:
- a first pipe (216), in which in use beverage to be carbonized and carbon dioxide flow; and
- a second pipe (214), in which in use beverage not to be carbonized flows;
- wherein at least one turbulence generation element (210a, 210b, 210c, 210d) is arranged in the first pipe
- **characterized in that** it comprises at least one control valve (224) configured to direct a fluid to the first pipe (216) and/or second pipe (214); and
**in that** the carbonization controller (150) is configured, when in a first operation mode of the flow-type carbonization apparatus, to switch the at least one control valve (224) such that beverage to be carbonized is directed to the first pipe (216) and beverage not to be carbonized is directed to the second pipe (214), and **in that** the carbonization controller is configured, when in a second operation mode, to switch the control valve (224) such as to direct a disinfection fluid through the second pipe (214); and
**in that** the first pipe (216) and second pipe (214) are in thermal communication such that in use heat from a fluid flowing in the second pipe (214) heats the first pipe (216).

2. The flow-type carbonization apparatus according to claim 1, wherein the first pipe (216) and second pipe (214) are arranged concentric.

3. The flow-type carbonization apparatus according to claim 1 or 2, wherein the first pipe (216) is arranged around the second pipe (214).

4. The flow-type carbonization apparatus according to any one of claims 1 to 3 wherein the at least one turbulence generation element (210a, 210b, 210c, 210d) reduces the cross section of the first pipe (216).

5. The flow-type carbonization apparatus according to any one of claims 1 to 4 **characterized by** at least one of the following:
- a plurality of turbulence generation elements (210a, 210b, 210c, 210d) is arranged apart serially in the flow direction of the beverage in the first pipe (216);
- a plurality of turbulence generation openings (212a, 212b, 212c, 212d) is arranged apart radially on the turbulence generation element (210a, 210b, 210c, 210d).

6. The flow-type carbonization apparatus according to any one of claims 1 to 5, wherein a plurality of turbulence generation openings (212a, 212b, 212c, 212d) is arranged apart around the circumference of the turbulence generation element.

7. The flow-type carbonization apparatus according to any one of claims 1 to 6, wherein the turbulence generation element (210a, 210b, 210c, 210d) has a generally circular cross section at its outer perimeter and wherein at least one turbulence generation opening (212a, 212b, 212c, 212d) is formed by at least one recess at the outer perimeter of the turbulence generation element (210a, 210b, 210c, 210d).

8. The flow-type carbonization apparatus according to claim 7, wherein the recess (212a, 212b, 212c, 212d) is formed by a flattened portion (220) of the generally circular cross section of the turbulence generation element (210a, 210b, 210c, 210d).

9. The flow-type carbonization apparatus according to claim 8, wherein the recess (220) is formed by a first wall (220) orthogonal to the radius of the first pipe (216) and at least one second wall (219) perpendicular to the first wall.

10. The flow-type carbonization apparatus according to any one of claims 1 to 9, wherein a plurality of turbulence generation elements (210a, 210b, 210c, 210d) is arranged in serial relationship forming turbulence chambers (222a, 222b, 222c, 222d) between the opposite turbulence generation element, the outer cylindrical wall of the second pipe (214) and the inner cylindrical wall of the first pipe (216).

11. The flow-type carbonization apparatus according to claim 10, **characterized by** at least one of the following:
- the distance in axial direction of the first pipe (216) between two turbulence generation elements (210a, 210b, 210c, 210d) arranged in serial relationship is at least 2 times of the thickness of the turbulence generation element (210a, 210b, 210c, 210d) in axial direction of the first pipe (216);
- the distance in axial direction of the first pipe (216) between two turbulence generation elements (210a, 210b, 210c, 210d) arranged in serial relationship ranges between approximately 2 to approximately 3 of times the thickness of the turbulence generation (210a, 210b, 210c, 210d) element in axial direction of the first pipe (216);
- the distance in axial direction of the first pipe (216) between two turbulence generation elements (210a, 210b, 210c, 210d) arranged in serial relationship is at least 2 times the difference of the inner diameter of the first pipe (216) and the outer diameter of the second pipe (214);
- the distance in axial direction of the first pipe (216) between two turbulence generation elements (210a, 210b, 210c, 210d) arranged in serial relationship is approximately 2 times to approximately 3 times the difference of the inner diameter of the first pipe (216) and the outer diameter of the second pipe (214);
- the width of the recess of the turbulence generation element (210a, 210b, 210c, 210d) orthogonal to the radius of the first pipe (216) ranges between approximately 75 % to approximately 125 % of the thickness of the turbulence generation element (210a, 210b, 210c, 210d) in axial direction of the first pipe (216);
- the maximum height of the recess in radial direction of the first pipe (216) ranges from approximately 0.5 % to approximately 1.5 % of the thickness of the turbulence generation element (210a, 210b, 210c, 210d) in axial direction of the first pipe (216).

12. A beverage dispenser (100) comprising the flow-type carbonization apparatus according to claims 1 to 11, further comprising
- at least one of a liquid flow valve and a liquid pump (106) adapted to control the flow of beverage through the flow-type carbonization apparatus;
- at least one of a gas valve (124, 126) and a gas pump adapted to control the flow of gas into a gas inlet portion (110) for supplying the beverage with carbon dioxide; and
- a controller (150) adapted to control the at least one of the liquid flow valve and the liquid pump (106) and the at least one of the gas valve (124, 126) and the gas pump, wherein the controller (150) controls the at least one of the liquid flow valve and the liquid pump and the at least one of the gas valve or gas pump such that gas is fed into the gas inlet portion during flow of the beverage through the flow-type carbonizing apparatus.

13. The beverage dispenser (100) according to 12, wherein the gas inlet portion comprises a first and second gas injector (124, 126) and wherein the carbonization controller (150) is adapted to control the first gas injector (124) and the second gas injector (126), wherein if a low quantity of gas shall be fed into the liquid, only the first gas injector is (124) activated, if a medium quantity of gas shall be fed into the liquid, only the second gas injector (126) is activated and if a high quantity of gas shall be fed into the liquid, the first gas injector (124) and the second gas injector (126) are activated.

14. The beverage dispenser (100) according to claim 13, further comprising a tempering device (128) arranged downstream of the gas injection portion and upstream of the turbulence device (200).

## Patentansprüche

1. Durchflusskarbonisierungsvorrichtung, aufweisend:
- eine Karbonisierungssteuerungseinrichtung (150);
- eine Durchflusskarbonisierungseinrichtung aufweisend:
- ein erstes Rohr (216), in dem im Einsatz zu karbonisierendes Getränk und Kohlendioxid strömen; und
- ein zweites Rohr, in dem im Einsatz nicht zu karbonisierendes Getränk strömt;
- wobei zumindest ein Turbulenzerzeugungselement (210a, 210b, 210c, 210d) in dem ersten Rohr angeordnet ist;
- **dadurch gekennzeichnet, dass** sie zumindest ein Steuerungsventil (224) aufweist, um ein Fluid zum ersten Rohr (216) und/oder zum zweiten Rohr (214) zu richten; und,
- dass die Karbonisierungssteuerungseinrichtung (150) eingerichtet ist, wenn sie sich in einer ersten Betriebsart der Durchflusskarbonisierungsvorrichtung befindet, das zumindest eine Steuerungsventil (224) zu schalten, dass das zu karbonisierendes Getränk in das erste Rohr (216) gerichtet wird und nicht zu karbonisierende Getränk in das zweite Rohr (214) gerichtet wird, und
- dass die Karbonisierenssteuerungseinrichtung dazu eingerichtet ist, wenn sie sich in einem zweiten Betriebsmodus befindet, das Steuerungsventil (224) so zu schalten, dass es ein Desinfektionfluid durch das zweite Rohr (214) leitet, und
- dass sich das erste Rohr (216) und das zweite Rohr (214) in thermischer Kommunikation befinden, so dass im Einsatz Wärme von einem durch das zweite Rohr (214) strömenden Fluid das erste Rohr (216) heizt.

2. Durchflusskarbonisierungsvorrichtung nach Anspruch 1, wobei das erste Rohr (216) und das zweite Rohr (214) konzentrisch angeordnet sind.

3. Durchflusskarbonisierungsvorrichtung nach Anspruch 1 oder 2, wobei das erste Rohr (216) um das zweite Rohr (214) angeordnet ist.

4. Durchflusskarbonisierungsvorrichtung nach einem der Ansprüche 1 bis 3, wobei das zumindest eine Turbulenzerzeugungselement (210a, 210b, 210c, 210d) den Querschnitt des ersten Rohrs (216) reduziert.

5. Durchflusskarbonisierungsvorrichtung nach einem der Ansprüche 1 bis 4, **gekennzeichnet durch** zumindest eines von Folgenden:
- eine Mehrzahl von Turbulenzerzeugungselementen (210a, 210b, 210c, 210d) sind voneinander beabstandet hintereinander in der Strömungsrichtung des Getränks im ersten Rohr (216) angeordnet;
- eine Mehrzahl von Turbulenzerzeugungsöffnungen (212a, 212b, 212c, 212d) ist voneinander beabstandet radial auf dem Turbulenzerzeugungselement (210a, 210b, 210c, 210d) angeordnet.

6. Durchflusskarbonisierungsvorrichtung nach einem der Ansprüche 1 bis 5, wobei eine Mehrzahl von Turbulenzerzeugungsöffnungen (212a, 212b, 212c, 212d) voneinander beabstandet um den Umfang des Turbulenzerzeugungselementes angeordnet ist.

7. Durchflusskarbonisierungsvorrichtung nach einem der Ansprüche 1 bis 6, wobei das Turbulenzerzeugungselement (210a, 210b, 210c, 210d) einen im Allgemeinen kreisförmigen Querschnitt an seinen Außenumfang aufweist und wobei zumindest eine Turbulenzerzeugungsöffnung (212a, 212b, 212c, 212d) durch zumindest eine Ausnehmung am äußeren Umfang des Turbulenzerzeugungselementes (210a, 210b, 210c, 210d) gebildet ist.

8. Durchflusskarbonisierungsvorrichtung nach Anspruch 7, wobei die Ausnehmung (212a, 212b, 212c, 212d) durch einen abgeflachten Bereich (220) des im Allgemeinen runden Querschnittes des Turbulenzerzeugungselementes (210a, 210b, 210c, 210d) gebildet ist.

9. Durchflusskarbonisierungsvorrichtung nach Anspruch 8, wobei die Ausnehmung (220) durch eine erste Wand (220) orthogonal zum Radius des ersten Rohrs (216) und durch zumindest eine zweite Wand (219) gebildet ist, die rechtwinklig zur ersten Wand ist.

10. Durchflusskarbonisierungsvorrichtung nach einem der Ansprüche 1 bis 9, wobei eine Mehrzahl von Turbulenzerzeugungselementen (210a, 210b, 210c, 210d) in serieller Beziehung angeordnet sind, um Turbulenzkammern (222a, 222b, 222c, 222d) zwischen entgegengesetzten Turbulenzerzeugungselementen, der äußeren zylindrischen Wand des zweiten Rohres (214) und der inneren zylindrischen Wand des ersten Rohres (216) zu bilden.

11. Durchflusskarbonisierungsvorrichtung nach Anspruch 10 **gekennzeichnet durch** zumindest einem von Folgenden:
- der Abstand in der axialen Richtung des ersten Rohrs (216) zwischen zwei Turbulenzerzeugungselementen (210a, 210b, 210c, 210d), die in serieller Beziehung angeordnet sind, ist zumindest das zweifache der Dicke des Turbulenzerzeugungselementes (210a, 210b, 210c, 210d) in der axialen Richtung des ersten Rohrs (216);
- der Abstand in der axialen Richtung des ersten Rohrs (216) zwischen zwei Turbulenzerzeugungselementen (210a, 210b, 210c, 210d), die in serieller Beziehung angeordnet sind, befindet sich im Bereich von etwa den zweifachen bis zu etwa dem dreifachen der Dicke des Turbulenzerzeugungselementes (210a, 210b, 210c, 210d) in der axialen Richtung des ersten Rohrs (216);
- der Abstand in axialer Richtung des ersten Rohrs (216) zwischen zwei Turbulenzerzeugungselementen (210a, 210b, 210c, 210d), die in serieller Beziehung angeordnet sind, ist zumindest zweimal die Differenz des inneren Durchmessers des ersten Rohrs (216) und des äußeren Durchmessers des zweiten Rohres (214);
- der Abstand in axialer Richtung des ersten Rohrs (216) zwischen zwei Turbulenzerzeugungselementen (210a, 210b, 210c, 210d), die in serieller Beziehung angeordnet sind, ist etwa das zweifache bis etwa dreifache der Differenz des inneren Durchmessers des ersten Rohrs (216) und des äußeren Durchmessers des zweiten Rohrs (214);
- die Breite der Ausnehmung des Turbulenzerzeugungselementes (210a, 210b, 210c, 210d) orthogonal zum Radius des ersten Rohrs (216) befindet sich in einem Bereich zwischen etwa 75 % bis etwa 125 % der Dicke des Turbulenzerzeugungselementes (210a, 210b, 210c, 210d) in der axialen Richtung des ersten Rohrs (216);
- die maximale Höhe der Ausnehmung in einer radialen Richtung des ersten Rohrs (216) befindet sich in einem Bereich von etwa 0,5 % bis etwa 1,5 % der Dicke des Turbulenzerzeugungselementes (210a, 210b, 210c, 210d) in axialer Richtung des ersten Rohrs (216).

12. Getränkespender mit der Durchflusskarbonisierungsvorrichtung nach einem der Ansprüche 1 bis 11, ferner aufweisend:
- zumindest einem aus einem Flüssigkeitsstromventil und einer Flüssigkeitspumpe (106), die dazu ausgebildet ist, die Strömung des Getränkes durch die Durchflusskarbonisierungsvorrichtung zu steuern;
- zumindest eines von einem Gasventil (124, 126) und einer Gaspumpe, die dazu ausgebildet sind, die Steuerung der Strömung des Gas in einen Gaseinlassbereich (110) zum Versorgen des Getränkes mit Kohlendioxid zu steuern; und
- eine Steuerungseinrichtung (150), die dazu ausgebildet ist, zumindest einem von Flüssigkeitsstromventil und Flüssigkeitspumpe (106) und zumindest einem von Gasventil (124,126) und Gaspumpe zu steuern, wobei die Steuerungseinrichtung (150) zumindest eines von Flüssigkeitsstromventil und Flüssigkeitspumpe und zumindest eines von Gasventil oder Gaspumpe so steuert, dass Gas in den Gaseinlassbereich während des Strömens des Getränkes durch die Durchflusskarbonisierungsvorrichtung zugeführt wird.

13. Getränkespender (100) nach Anspruch 12, wobei der Gaseinlassbereich einen ersten und zweiten Gasinjektor (124, 126) aufweist und wobei die Karbonisierungssteuerungseinrichtung (150) dazu ausgebildet ist, den ersten Gasinjektor (124) und den zweiten Gasinjektior (126) zu steuern, wobei, falls eine niedrige Menge an Gas der Flüssigkeit zugeführt werden soll, lediglich der erste Gasinjektor (124) aktiviert wird, falls eine mittlere Menge an Gas der Flüssigkeit zugeführt werden soll, lediglich der zweite Gasinjektor (126) aktiviert wird, und falls eine hohe Menge an Gas der Flüssigkeit zugeführt werden soll, der erste Gasinjektor (124) und der zweite Gasinjektor (126) aktiviert werden.

14. Getränkespender (100) nach Anspruch 13, ferner aufweisend eine Temperiereinrichtung (128) die strömungsabwärts des Gasinjektorbereichs und strömungsaufwärts der Turbulenzeinrichtung (200) angeordnet ist.

## Revendications

1. Appareil de carbonisation du type flux, comprenant :
- un contrôleur de carbonisation (150) ; et
- un dispositif de carbonisation du type flux incluant :
- un premier tube (216) dans lequel, en utilisation, une boisson qui doit être carbonisée et du dioxyde de carbone s'écoulent ; et
- un second tube (214) dans lequel, en utilisation, une boisson qui ne doit pas être carbonisée s'écoule ;
dans lequel au moins un élément de génération de turbulence (210a, 210b, 210c, 210d) est agencé à l'intérieur du premier tube ;
**caractérisé en ce que** :
il comprend au moins une soupape de commande (224) qui est configurée pour diriger un fluide sur le premier tube (216) et/ou sur le second tube (214) ; et **en ce que** :
le contrôleur de carbonisation (150) est configuré, dans un premier mode de fonctionnement de l'appareil de carbonisation du type flux, pour commuter l'au moins une soupape de commande (224) de telle sorte que la boisson qui doit être carbonisée soit dirigée sur le premier tube (216) et que la boisson qui ne doit pas être carbonisée soit dirigée sur le second tube (214) ; et **en ce que** :
le contrôleur de carbonisation est configuré, dans un second mode de fonctionnement, pour commuter l'au moins une soupape de commande (224) de manière à diriger un fluide de désinfection au travers du second tube (214) ; et **en ce que** :
le premier tube (216) et le second tube (214) sont en communication thermique de telle sorte que, en utilisation, de la chaleur en provenance d'un fluide qui s'écoule à l'intérieur du second tube (214) chauffe le premier tube (216).

2. Appareil de carbonisation du type flux selon la revendication 1, dans lequel le premier tube (216) et le second tube (214) sont agencés de façon concentrique.

3. Appareil de carbonisation du type flux selon la revendication 1 ou 2, dans lequel le premier tube (216) est agencé autour du second tube (214).

4. Appareil de carbonisation du type flux selon l'une quelconque des revendications 1 à 3, dans lequel l'au moins un élément de génération de turbulence (210a, 210b, 210c, 210d) réduit la section en coupe transversale du premier tube (216).

5. Appareil de carbonisation du type flux selon l'une quelconque des revendications 1 à 4, **caractérisé par** au moins l'une des assertions qui suivent :
- les éléments d'une pluralité d'éléments de génération de turbulence (210a, 210b, 210c, 210d) sont agencés de telle sorte qu'ils soient espacés les uns des autres en série dans la direction d'écoulement de la boisson dans le premier tube (216) ; et
- les ouvertures d'une pluralité d'ouvertures de génération de turbulence (212a, 212b, 212c, 212d) sont agencées de telle sorte qu'elles soient espacées les unes des autres radialement sur l'élément de génération de turbulence (210a, 210b, 210c, 210d).

6. Appareil de carbonisation du type flux selon l'une quelconque des revendications 1 à 5, dans lequel les ouvertures d'une pluralité d'ouvertures de génération de turbulence (212a, 212b, 212c, 212d) sont agencées de telle sorte qu'elles soient espacées les unes des autres le long de la circonférence de l'élément de génération de turbulence.

7. Appareil de carbonisation du type flux selon l'une quelconque des revendications 1 à 6, dans lequel l'élément de génération de turbulence (210a, 210b, 210c, 210d) présente une section en coupe transversale de forme générale circulaire au niveau de son périmètre externe et dans lequel au moins une ouverture de génération de turbulence (212a, 212b, 212c, 212d) est formée par au moins un évidement au niveau du périmètre externe de l'élément de génération de turbulence (210a, 210b, 210c, 210d).

8. Appareil de carbonisation du type flux selon la revendication 7, dans lequel l'évidement (212a, 212b, 212c, 212d) est formé par une partie aplanie (220) de la section en coupe transversale de forme générale circulaire de l'élément de génération de turbulence (210a, 210b, 210c, 210d).

9. Appareil de carbonisation du type flux selon la revendication 8, dans lequel l'évidement (220) est formé par une première paroi (220) qui est orthogonale par rapport au rayon du premier tube (216) et par au moins une seconde paroi (219) qui est perpendiculaire à la première paroi.

10. Appareil de carbonisation du type flux selon l'une quelconque des revendications 1 à 9, dans lequel les éléments d'une pluralité d'éléments de génération de turbulence (210a, 210b, 210c, 210d) sont agencés selon une relation série en formant des chambres de turbulence (222a, 222b, 222c, 222d) entre l'élément de génération de turbulence opposé, la paroi cylindrique externe du second tube (214) et la paroi cylindrique interne du premier tube (216).

11. Appareil de carbonisation du type flux selon la revendication 10, **caractérisé par** au moins l'une des assertions qui suivent :
- la distance dans une direction axiale du premier tube (216) entre deux éléments de génération de turbulence (210a, 210b, 210c, 210d) qui sont agencés selon une relation série est au moins égale à 2 fois l'épaisseur de l'élément de génération de turbulence (210a, 210b, 210c, 210d) dans une direction axiale du premier tube (216) ;
- la distance dans une direction axiale du premier tube (216) entre deux éléments de génération de turbulence (210a, 210b, 210c, 210d) qui sont agencés selon une relation série s'inscrit à l'intérieur d'une plage entre approximativement 2 fois et approximativement 3 fois l'épaisseur de l'élément de génération de turbulence (210a, 210b, 210c, 210d) dans une direction axiale du premier tube (216) ;
- la distance dans une direction axiale du premier tube (216) entre deux éléments de génération de turbulence (210a, 210b, 210c, 210d) qui sont agencés selon une relation série est au moins égale à 2 fois la différence entre le diamètre interne du premier tube (216) et le diamètre externe du second tube (214) ;
- la distance dans une direction axiale du premier tube (216) entre deux éléments de génération de turbulence (210a, 210b, 210c, 210d) qui sont agencés selon une relation série est égale à approximativement 2 fois à approximativement 3 fois la différence entre le diamètre interne du premier tube (216) et le diamètre externe du second tube (214) ;
- la largeur de l'évidement de l'élément de génération de turbulence (210a, 210b, 210c, 210d) qui est orthogonal par rapport au rayon du premier tube (216) s'inscrit à l'intérieur d'une plage entre approximativement 75 % et approximativement 125 % de l'épaisseur de l'élément de génération de turbulence (210a, 210b, 210c, 210d) dans une direction axiale du premier tube (216) ; et
- la hauteur maximum de l'évidement dans une direction radiale du premier tube (216) s'inscrit à l'intérieur d'une plage entre approximativement 0,5 % et approximativement 1,5 % de l'épaisseur de l'élément de génération de turbulence (210a, 210b, 210c, 210d) dans une direction axiale du premier tube (216).

12. Distributeur de boissons (100) comprenant l'appareil de carbonisation du type flux selon les revendications 1 à 11, comprenant en outre :
- au moins un élément constitutif pris parmi une soupape à écoulement de liquide et une pompe à liquide (106) dont la fonction consiste à contrôler l'écoulement d'une boisson au travers de l'appareil de carbonisation du type flux ;
- au moins un élément constitutif pris parmi une soupape à gaz (124, 126) et une pompe à gaz dont la fonction consiste à contrôler l'écoulement d'un gaz à l'intérieur d'une partie d'entrée de gaz (110) pour alimenter la boisson en dioxyde de carbone ; et
- un contrôleur (150) dont la fonction consiste à commander l'au moins un élément constitutif pris parmi la soupape à écoulement de liquide et la pompe à liquide (106) et l'au moins un élément constitutif pris parmi la soupape à gaz (124, 126) et la pompe à gaz ;
dans lequel le contrôleur (150) commande l'au moins un élément constitutif pris parmi la soupape à écoulement de liquide et la pompe à liquide et l'au moins un élément constitutif pris parmi la soupape à gaz et la pompe à gaz de telle sorte que du gaz soit alimenté à l'intérieur de la partie d'entrée de gaz pendant l'écoulement de la boisson au travers de l'appareil de carbonisation du type flux.

13. Distributeur de boissons (100) selon la revendication 12, dans lequel la partie d'entrée de gaz comprend des premier et second injecteurs de gaz (124, 126), et dans lequel le contrôleur de carbonisation (150) a pour fonction de commander le premier injecteur de gaz (124) et le second injecteur de gaz (126), dans lequel, si une quantité faible de gaz devait être alimentée à l'intérieur du liquide, seulement le premier injecteur de gaz (124) devrait être activé, si une quantité intermédiaire de gaz devait être alimentée à l'intérieur du liquide, seulement le second injecteur de gaz (126) devrait être activé et si une quantité élevée de gaz devait être alimentée à l'intérieur du liquide, le premier injecteur de gaz (124) et le second injecteur de gaz (126) devraient être activés.

14. Distributeur de boissons (100) selon la revendication 13, comprenant en outre un dispositif de régulation thermique (128) qui est agencé en aval de la partie d'injection de gaz et en amont du dispositif de turbulence (200).
